# EUROPÄISCHE PATENTSCHRIFT

(11) **EP 3 285 845 B1**
(45) Veröffentlichungstag und Bekanntmachung des Hinweises auf die Patenterteilung: **28.05.2025**
(21) Anmeldenummer: 16731014.3
(22) Anmeldetag: 21.04.2016
(51) Int. Cl.: A61M 25/00

(54) **KANÜLE**
CANNULA
CANULE

(30) Priorität: 21.04.2015 DE 102015005002
(43) Veröffentlichungstag der Anmeldung: 28.02.2018
(73) Patentinhaber: Xenios AG, 74076 Heilbronn (DE)
(72) Erfinder: SIMUNDIC, Ivo, 73240 Wendlingen (DE)
(74) Vertreter: Graf von Stosch, Andreas
(86) Internationale Anmeldenummer: PCT/DE2016/000172
(87) Internationale Veröffentlichungsnummer: WO 2016/169547

(56) Entgegenhaltungen:
- WO-A1-99/37351
- WO-A1-99/37352
- WO-A2-00/38591
- WO-A2-2014/113257
- DE-A1- 19 605 864
- DE-B3- 10 336 902
- US-A- 5 171 218
- US-A1- 2011 152 741
- US-A1- 2013 090 608
- US-A1- 2014 336 559

## Beschreibung

Die Erfindung betrifft eine Kanüle mit einer Spitze und einem Auslass. Derartige Kanülen werden insbesondere als arterielle Kanüle verwendet. Sie werden in verschiedenen Durchmessern und Längen angeboten.

Eine Kanüle mit einem Ventil ist aus der DE 196 05 864 A1 bekannt. Dabei sind am Ende der Kanüle mehrere spiralförmige Schlitze ausgebildet, die sich als Reaktion auf Druckänderungen im Lumen der Kanüle verbreitern oder verengen, um die Fluidgeschwindigkeit zu reduzieren.

Siehe weiterhin, zum Beispiel, WO 99/37351 A1.

Der Erfindung liegt die Aufgabe zugrunde, eine derartige Kanüle weiter zu entwickeln.

Diese Aufgabe wird dadurch gelöst, dass die erfindungsgemäße Kanüle gemäß dem unabhängigen Anspruch 1 eine Reduktion des Innendurchmessers aufweist und ein Ventil im Bereich der Reduktion des Durchmessers angeordnet ist.

Die Reduktion des Innendurchmessers kann exemplarisch und nicht erfindungsgemäß als eine konisch zulaufende Spitze ausgebildet sein. In diesem Fall ist es vorteilhaft, wenn das konische Ende der Spitze eine Länge hat, die kürzer ist als der Innendurchmesser des daran anschließenden Bereichs der Kanüle.

Die Reduktion des Innendurchmessers gemäß der erfindungsgemäßen arteriellen Kanüle ist als Kaliberverengung zwischen zwei zylindrischen Kanülenabschnitten mit unterschiedlichen Durchmessern ausgebildet.

Die Reduktion des Innendurchmessers liegt 25 % - 40 %, vorzugsweise etwa 30 % - 35 % der Einführlänge von der Spitze der Kanüle entfernt. Bei einem vorteilhaften Ausführungsbeispiel beginnt die Verjüngung nach etwa zwei Drittel der Kanülen-, bzw. Einführlänge.

Eine bevorzugte Einführlänge der Kanüle liegt bei 20 - 50 cm, vorzugsweise zwischen 28 und 45 cm. Dies ermöglicht verschiedene Platzierungsvarianten, je nach Länge. Eine kurze Einführlänge ermöglicht eine Platzierung unterhalb der Arteria renalis und eine lange Einführlänge ermöglicht eine Platzierung oberhalb oder auf der Höhe der Arteria renalis.

Je nach Reduktion des Durchmessers entstehen unterschiedliche Kalibersprünge, die bei einem Delta von 2 Fr - 4 Fr liegen. Praxisrelevante Beispiele liegen bei 15 Fr auf 13 Fr, 17 Fr auf 15 Fr, 17 Fr auf 13 Fr, 19 Fr auf 17 Fr und 19 Fr auf 15 Fr.

Eine derartige Reduktion des Innendurchmessers einer Kanüle führt zu einem Jetstrom an der Kanülenspitze, in der kurzen Einführlänge oder im durchmesserreduzierten Kanülenbereich.

Die erfindungsgemäße Kanüle sieht vor, dass sie seitliche Löcher aufweist und die Reduktion des Durchmessers zwischen den seitlichen Löchern und der Spitze der Kanüle angeordnet ist. Durch die Anordnung derartiger Löcher in der Kanüle wird die Perfusion der unteren Extremitäten gesichert und die Nachlast verringert.

Die Seitenlöcher können entweder an allen vier Quadranten einer Kanüle vorgesehen werden, sodass mehrere Löcher auf der gleichen Querschnittsebene der Kanüle liegen. Außerdem können mehrere Löcher, wie beispielsweise ein bis drei Löcher in Reihe hintereinander angeordnet werden.

Eine andere Ausführungsform sieht vor, dass in zwei Quadranten (180°) oder drei Quadranten (120°) jeweils ein bis drei Löcher hintereinander angeordnet werden. Es können jedoch auch zwei Bereiche mit hintereinander je drei Löchern vorgesehen sein oder Löcher, die um 90° zueinander versetzt angeordnet sind.

Die erfindungsgemäße Kanüle weist ein Ventil auf, das im Bereich der Reduktion des Durchmessers angeordnet ist. Ein bevorzugtes Ausführungsbeispiel sieht eine Kanüle mit einem Ventil vor, das mindestens eine und vorzugsweise mehrere Klappen aufweist.

Ein derartiger Ventilmechanismus orientiert sich am Prinzip der Aortenklappe mit beispielsweise drei Segeln. Vorzugsweise ist die Klappenbasis am Übergang zum Kalibersprung angebracht. Durch den stärkeren Fluss während der Pumpenbeschleunigung öffnet die Klappe. Das Verhältnis von relativer Klappenöffnung zum Volumenstrom kann durch die Positionierung und Ausbildung der Klappe eingestellt werden.

Gemäß der erfindungsgemäßen Ausbildung liegt das Ventil zwischen der Öffnung an der Kanülenspitze und in der Kanüle angebrachten seitlichen Löchern. Somit erfolgt ein Teilstrom der Perfusion durch das Kanülenzentrum zur Kanülenspitze und ein anderer Teilstrom erfolgt durch die an der Kanülenwandung angebrachten Löcher. Das Ventil, das zwischen den seitlichen Löchern und der Kanülenspitze liegt, kann dadurch diese zwei Teilströme variieren. Bei verengtem Ventil erhöht sich der Volumenstrom in den Seitenlöchern und bei geöffnetem Ventil erhöht sich der Volumenstrom zur Kanülenspitze.

Ein bevorzugtes Ausführungsbeispiel sieht vor, dass das Ventil mindestens eine und vorzugsweise mehrere Klappen aufweist. Diese Klappen können innerhalb der Kanüle angeordnet sein, um den Durchfluss innerhalb der Kanüle zur Kanülenspitze einzuschränken. Dabei ist es vorteilhaft, wenn mindestens eine Klappe einen Federmechanismus aufweist. Dieser Federmechanismus kann durch eine Feder oder durch die Materialwahl und Ausbildung der Klappen erzielt werden.

Beim Einsatz der Kanüle kann während der Phase des geringen Durchflusses durch die Kanüle, beispielsweise während der Herzsystole, die Federkraft des Ventils die Kanüle schließen oder den Durchfluss reduzieren. Dabei wirkt die Kraft des Volumenstroms gegen die Federkraft des Ventils. Wenn die Kraft des Volumenstroms geringer ist als die Federkraft des Ventils, schließt sich das Ventil. Dies hat zur Folge, dass, sofern Seitenlöcher angeordnet sind, der Blutfluss größtenteils durch die Seitenlöcher geleitet wird und so beispielsweise die unteren Extremitäten versorgt. Dies geschieht beispielsweise mit der Herzsystole während eines cardiogenen Schocks, da diese zu schwach ist, um das Ventil zu öffnen, wodurch die unteren Extremitäten besser mit Blut versorgt werden. Je nach Anordnung der Seitenlöcher kann in dieser Situation auch die Arteria renalis versorgt werden.

Als Mechanismus zum Verschließen des Ventils kann ein passiver Mechanismus vorgesehen werden, bei dem durch die Materialsteifheit der Klappe bei reduziertem Volumenstrom der Durchfluss durch das Ventil reduziert wird. Alternativ oder kumulativ kann ein Federmechanismus an den äußeren Rändern der Klappensegel vorgesehen sein.

Ein weiteres Ausführungsbeispiel der Erfindung sieht vor, dass die Kanüle an ihrer Innenseite mindestens bereichsweise eine spiralförmige Struktur aufweist. Durch eine derartige Struktur kann die innerhalb der Kanüle fließende Flüssigkeit einen Drall erhalten, der eine Stabilisierung des Flusses bewirkt. Dies ist insbesondere in Verbindung mit der Verjüngung, den Öffnungen oder dem Ventil von Vorteil, da jegliche Veränderung an der Innenseite der Kanüle den stabilen Volumenstrom beeinflusst.

Eine spiralförmige Struktur an der Innenseite der Kanüle kann beispielsweise durch eine spiralförmige Anordnung der Löcher innerhalb der Kanüle erzielt werden. Alternativ oder Kumulativ wird jedoch vorgesehen, dass nach dem Prinzip des Gewehrlaufes eine Wendelprägung an der Innenseite der Kanüle vorgesehen wird. Nach einem ersten Ausführungsbeispiel ist die Struktur als Erhebung ausgebildet. Hierzu wird beispielsweise eine konvex in das Volumeninnere hineinragende spiralförmige ein- oder mehrgängige Struktur vorgesehen. Eine derartige Struktur kann in die Kanüleninnenwandung eingearbeitet oder auf sie aufgetragen werden. Dabei können auch Mischformen zwischen konvexen und konkaven Kanülenbereichen vorgesehen werden, die durch Auftrag und Abtrag an der Kanüleninnenwandung oder durch eine entsprechende Ausbildung der Kanüleninnenwandung erzielt werden.

Die Struktur kann dabei innerhalb der Kanüle sich über die gesamte Kanülenlänge oder nur über einen Teil der Kanülenlänge erstrecken. Beispielsweise kann die Struktur nur in Flussrichtung im letzten Drittel der Einführlänge vorgesehen werden, um dem in der Kanüle fließenden Medium nur dort einen Drall aufzugeben.

Die Steigung der spiralförmigen Struktur bewirkt den jeweiligen Drall. Die Steigung der Struktur kann als Sinus α (Kanülenlänge durch Kanülendurchmesser) oder als Sinus α des reziproken Wertes angegeben werden. Daraus ergibt sich eine Steigung = Sinus α (Kanülenlänge durch Durchmesser) oder eine Steigung = Sinus α (1 durch Kanülenlänge durch Durchmesser).

Eine spezielle Ausführungsvariante sieht vor, dass die Struktur von einer Drahtverstärkung der Kanüle gebildet ist. Eine innerhalb der Kanüle vorgesehene Drahtverstärkung kann spiralförmig ausgebildet sein und zu einer entsprechenden spiralförmigen Struktur innerhalb der Kanüle führen, die einen Drall innerhalb der Kanüle bewirkt.

Ausführungsbeispiele erfindungsgemäßer Kanülen sind in der Zeichnung dargestellt und werden im Folgenden näher erläutert. Es zeigt
- Figur 1: eine Seitenansicht einer Kanüle mit Verjüngung und verlängerter Spitze,
- Figur 2: eine Seitenansicht einer nicht erfindungsgemäßen Kanüle mit kegelförmiger Spitze,
- Figur 3: eine Seitenansicht einer Kanüle mit mehreren Löchern am Umfang,
- Figur 4: eine Seitenansicht einer Kanüle mit am Umfang versetzt zueinander angeordneten Löchern,
- Figur 5: schematisch die Funktion eines offenen Ventils in einer teilweise geschnittenen Seitenansicht,
- Figur 6: schematisch eine Vorderansicht des in Figur 5 gezeigten Ventils,
- Figur 7: schematisch eine Seitenansicht eines teilweise geschlossenen Ventils,
- Figur 8: schematisch eine Vorderansicht des in Figur 7 gezeigten Ventils,
- Figur 9: schematisch einen Schnitt durch eine Kanüle mit einer konvexen spiralförmigen Erhebung,
- Figur 10: schematisch einen Schnitt durch eine Kanüle mit zwei gegenüberliegenden spiralförmigen Erhebungen und
- Figur 11: schematisch einen Schnitt durch eine Kanüle mit vier konkav ausgebildeten spiralförmigen Vertiefungen.

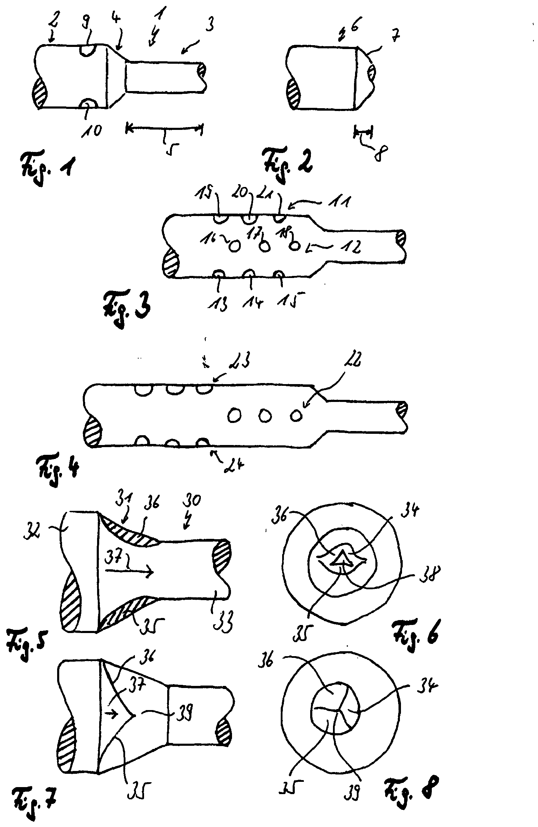

Die in Figur 1 gezeigte erfindungsgemäße Kanüle 1 hat einen zylinderförmigen Grundkörper 2, eine zylinderförmige Kanülenspitze 3 und dazwischen als Durchmesserreduktion 4 eine konische Verjüngung, die einen Übergang zwischen dem zylinderförmigen Grundkörper 2 mit dem größeren Durchmesser zur zylinderförmigen Spitze 3 mit dem geringeren Durchmesser bildet. Die Position der Verjüngung 4 ist derart gewählt, dass die Länge 5 zwei Drittel der Kanülen-, bzw. Einführlänge beträgt.

Die Figur 2 zeigt ein alternatives vorderes Ende einer nicht erfindungsgemäßen Kanüle 6, bei dem eine konische Verengung 7 das vordere Ende der Kanüle 6 bildet, sodass die Kanüle 6 eine konische Kanülenspitze mit der Länge 8 aufweist.

Innerhalb der Kanüle und im Grundkörper 2 der Kanüle mit größerem Durchmesser sind Löcher 9, 10 vorgesehen. Die Figur 3 zeigt eine Anordnung von Löchern, die in Längsrichtung der Kanüle jeweils um 90° versetzt sind. Im Ausführungsbeispiel ergeben sich somit vier jeweils leicht zueinander versetzte Lochreihen 11, 12, mit jeweils drei Löchern 13 - 15, 16 - 18 und 19 - 21.

In dem in Figur 4 gezeigten Ausführungsbeispiel sind jeweils die gesamten Lochreihen 22, 23, 24 versetzt zueinander angeordnet. Im Ausführungsbeispiel haben die Lochreihen jeweils drei Löcher. Es können jedoch auch zwei oder mehr Löcher vorgesehen sein.

Bei dem in den Figuren 5 - 8 gezeigten Ventil 30 sind im Verjüngungsbereich 31 zwischen einem Kanülenbereich 32 mit größerem Durchmesser und einem Kanülenbereich 33 mit verringertem Durchmesser drei Klappensegel 34, 35, 36 vorgesehen, die im Verjüngungsbereich 31 an der Innenwandung der Kanüle befestigt sind. Diese Klappensegel 34 - 36 werden durch einen Volumenstrom 37 geöffnet, sodass eine Öffnung 38 entsteht, durch die eine Flüssigkeit vom Kanülenbereich 32 zum Kanülenbereich 33 fließen kann.

Sofern der Druck des Volumenstroms 37, wie in den Figuren 7 und 8 gezeigt, zurückgeht, werden die Klappensegel 34 - 36 durch ihre Materialsteifheit nach innen gedrängt, sodass sie die Öffnung 38 verschließen, indem die Klappensegel 34 - 36 am Punkt 39 aneinander anliegen und den Durchfluss behindern oder stoppen.

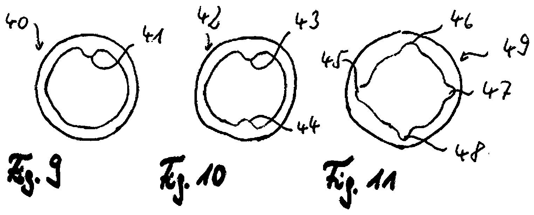

Verschiedene Varianten zum Aufbringen eines Dralls innerhalb der Kanüle sind in den Figuren 9 - 11 dargestellt. Dies sind nur schematisch dargestellte Ausführungsvarianten, die die Möglichkeit des Aufbringens eines Dralls innerhalb der Kanüle andeuten sollen. Figur 9 zeigt eine Kanüle 40 mit einer radial nach innen sich auswölbenden Erhebung 41, die sich spiralförmig innerhalb der Kanüle erstreckt. Eine zweigängige Ausführungsform ist in Figur 10 gezeigt. Hier ist innerhalb der Kanüle 42 eine erste Erhebung 43 und der Erhebung 43 gegenüberliegend eine zweite Erhebung 44 vorgesehen. Beide Erhebungen sind Teil einer doppelgängigen, innerhalb der Kanüle 42 ausgebildeten Spirale.

In Figur 11 ist am Beispiel von vier Vertiefungen dargestellt, wie auch mittels Vertiefungen 45 - 48 innerhalb einer Kanüle 49 ein Drall auf eine in der Kanüle geführte Flüssigkeit ausgeübt werden kann.

## Patentansprüche

1. Arterielle Kanüle (1) mit einer Spitze (3) und einem Auslass, wobei die Kanüle eine Reduktion (4) des Innendurchmessers, welche als Kaliberverengung zwischen zwei zylindrischen Kanülenabschnitten mit unterschiedlichen Durchmessern ausgebildet ist, und ein Ventil (30) aufweist, das im Bereich der Reduktion (4) des Durchmessers angeordnet ist, *wobei* die Kanüle seitliche Löcher (13 - 18) aufweist und die Reduktion des Durchmessers zwischen den seitlichen Löchern und der Spitze (3) der Kanüle angeordnet ist
**dadurch gekennzeichnet, dass**
die Reduktion (4) des Innendurchmessers 25 % bis 40 % der Einführlänge von der Spitze (3) der Kanüle (1) entfernt und bei einer Reduktion von 2 bis 4 Fr liegt.

2. Arterielle Kanüle nach Anspruch 1, ***dadurch gekennzeichnet, dass*** das Ventil (30) mindestens eine und vorzugsweise mehrere Klappen (34 - 36) aufweist.

3. Arterielle Kanüle nach Anspruch 2, ***dadurch gekennzeichnet, dass*** die mindestens eine Klappe (34 - 36) einen Federmechanismus aufweist.

4. Arterielle Kanüle nach Anspruch 1 oder 2, ***dadurch gekennzeichnet, dass*** eine Basis einer Klappe am Übergang zum Kalibersprung angeordnet ist.

5. Arterielle Kanüle nach einem der vorhergehenden Ansprüche, ***dadurch gekennzeichnet, dass*** die Reduktion (4) des Innendurchmessers als eine konisch zulaufende Spitze (7) ausgebildet ist.

6. Arterielle Kanüle nach Anspruch 5, **dadurch *gekennzeichnet,***
***dass*** das konische Ende der Spitze (3) eine Länge (5) hat, die kürzer ist als der Innendurchmesser des daran anschließenden Bereichs der Kanüle.

7. Arterielle Kanüle nach einem der vorstehenden Ansprüche, ***dadurch gekennzeichnet, dass*** die Reduktion des Innendurchmessers 30 % bis 35 % der Einführlänge von der Spitze der Kanüle entfernt liegt.

8. Arterielle Kanüle nach einem der vorhergehenden Ansprüche, ***dadurch gekennzeichnet, dass*** sie eine Einführlänge von 20 bis 50 cm, vorzugsweise von 28 bis 45 cm aufweist.

9. Arterielle Kanüle nach einem der vorhergehenden Ansprüche, ***dadurch gekennzeichnet, dass*** die Reduktion (4) des Durchmessers einen Kalibersprung von 15 Fr auf 13 Fr, 17 Fr auf 15 Fr, 17 Fr auf 13 Fr, 19 Fr auf 17 Fr oder 19 Fr auf 15 Fr umfasst.

10. Arterielle Kanüle nach einem der vorhergehenden Ansprüche, ***dadurch gekennzeichnet, dass*** sie an ihrer Innenseite zumindest bereichsweise eine spiralförmige Struktur (41) aufweist.

11. Arterielle Kanüle nach Anspruch 10, ***dadurch gekennzeichnet, dass*** die Struktur (41) eine Erhebung ist.

12. Arterielle Kanüle nach Anspruch 10, **dadurch *gekennzeichnet, dass*** die Struktur (45 - 48) eine Vertiefung ist.

13. Arterielle Kanüle nach einem der Ansprüche 10 bis 12, ***dadurch gekennzeichnet, dass*** die Struktur (43, 44) mehrgängig ist.

14. Arterielle Kanüle nach einem der Ansprüche 10 bis 13, ***dadurch gekennzeichnet, dass*** die Struktur von einer Drahtverstärkung der Kanüle gebildet ist.

## Claims

1. Arterial cannula (1) with a tip (3) and an outlet, the cannula having a reduction (4) of the internal diameter, which is formed as a caliber narrowing between two cylindrical cannula sections with different diameters, and a valve (30) which is arranged in the region of the reduction (4) of the diameter, wherein the cannula has lateral holes (13-18) and wherein the reduction of the diameter is arranged between the lateral holes and the tip (3) of the cannula **characterized in that**
the reduction (4) of the internal diameter is 25 % to 40 % of the insertion length away from the tip (3) of the cannula (1) and is at a reduction of 2 to 4 Fr.

2. Arterial cannula according to claim 1, ***characterized in that** the* valve (30) has at least one and preferably several flaps (34 - 36).

3. Arterial cannula according to claim 2, ***characterized in that*** the at least one flap (34 - 36) has a spring mechanism.

4. Arterial cannula according to claim 1 or 2, ***characterized in that*** a base of a flap is arranged at the transition zone to the caliber change.

5. Arterial cannula according to one of the preceding claims, ***characterized in that*** the reduction (4) of the internal diameter is designed as a conically tapering tip (7).

6. Arterial cannula according to claim 5, ***characterized in that** the* conical end of the tip (3) has a length (5) which is shorter than the internal diameter of the adjoining section of the cannula.

7. Arterial cannula according to one of the preceding claims, ***characterized in that*** the reduction of the internal diameter is 30 % to 35 % of the insertion length away from the tip of the cannula.

8. Arterial cannula according to one of the preceding claims, ***characterized in that*** it has an insertion length of 20 to 50 cm, preferably 28 to 45 cm.

9. Arterial cannula according to one of the preceding claims, ***characterized in that*** the reduction (4) in diameter comprises a change in caliber from 15 Fr to 13 Fr, 17 Fr to 15 Fr, 17 Fr to 13 Fr, 19 Fr to 17 Fr or 19 Fr to 15 Fr.

10. Arterial cannula according to one of the preceding claims, ***characterized in that*** it has a spiral-shaped structure (41) on its inner side, at least in certain sections.

11. Arterial cannula according to claim 10, ***characterized in that*** the structure (41) is an elevation.

12. Arterial cannula according to claim 10, ***characterized in that*** the structure (45 - 48) is a recession.

13. Arterial cannula according to any one of claims 10 to 12, ***characterized in that** the* structure (43, 44) is multi-threaded.

14. Arterial cannula according to any one of claims 10 to 13, ***characterized in that*** the structure is formed by a wire reinforcement of the cannula.

## Revendications

1. Canule artérielle (1) présentant une pointe (3) et une sortie, la canule présentant une réduction (4) du diamètre interne, qui est conçue comme un rétrécissement de calibre entre deux sections cylindriques de canule présentant des diamètres différents, et une vanne (30), qui est agencée dans la zone de la réduction (4) du diamètre, la canule présentant des trous (13-18) latéraux et la réduction du diamètre étant agencée entre les trous latéraux et la pointe (3) de la canule,
**caractérisée en ce que**
la réduction (4) du diamètre interne se situe à une distance de 25% à 40% de la longueur d'introduction par rapport à la pointe (3) de la canule (1) et à une réduction de 2 à 4 Fr.

2. Canule artérielle selon la revendication 1, **caractérisée en ce que** la vanne (30) présente au moins un et de préférence plusieurs clapets (34-36).

3. Canule artérielle selon la revendication 2, **caractérisée en ce que** ledit au moins un clapet (34-36) présente un mécanisme à ressort.

4. Canule artérielle selon la revendication 1 ou 2, **caractérisée en ce qu'**une base d'un clapet est agencée au niveau de la transition avec le saut de calibre.

5. Canule artérielle selon l'une des revendications précédentes, **caractérisée en ce que** la réduction (4) du diamètre interne est réalisée comme une pointe (7) s'effilant de manière conique.

6. Canule artérielle selon la revendication 5, **caractérisée en ce que** l'extrémité conique de la pointe (3) présente une longueur (5) qui est plus courte que le diamètre interne de la zone de la canule qui y est raccordée.

7. Canule artérielle selon l'une des revendications précédentes, **caractérisée en ce que** la réduction du diamètre interne se situe à une distance de 30 % à 35 % de la longueur d'introduction par rapport à la pointe de la canule.

8. Canule artérielle selon l'une des revendications précédentes, **caractérisée en ce qu'**elle présente une longueur d'introduction de 20 à 50 cm, de préférence de 28 à 45 cm.

9. Canule artérielle selon l'une des revendications précédentes, **caractérisée en ce que** la réduction (4) du diamètre comprend un saut de calibre de 15 Fr à 13 Fr, de 17 Fr à 15 Fr, de 17 Fr à 13 Fr, de 19 Fr à 17 Fr ou de 19 Fr à 15 Fr.

10. Canule artérielle selon l'une des revendications précédentes, **caractérisée en ce qu'**elle présente sur sa face interne, au moins sur une zone, une structure (41) en forme de spirale.

11. Canule artérielle selon la revendication 10, **caractérisée en ce que** la structure (41) est une élévation.

12. Canule artérielle selon la revendication 10, **caractérisée en ce que** la structure (45-48) est un creux.

13. Canule artérielle selon l'une des revendications 10 à 12, **caractérisée en ce que** la structure (43, 44) est à pas multiples.

14. Canule artérielle selon l'une des revendications 10 à 13, **caractérisée en ce que** la structure est formée par un renforcement en fil de la canule.
